# EUROPEAN PATENT APPLICATION

(11) **EP 4 046 627 A1**
(43) Date of publication of application: **24.08.2022**
(21) Application number: 20877751.6
(22) Date of filing: 16.10.2020
(51) Int. Cl.: A61K 9/127, A61K 31/47, A61K 9/19, A61P 33/06

(54) **DECOQUINATE LIPOSOME, PREPARATION METHOD THEREFOR AND USE THEREOF**

(30) Priority: 18.10.2019 CN 201910995758
(71) Applicant: CAS Lamvac (Guangzhou) Biomedical Technology Co., Ltd., China-Singapore Guangzhou Knowledge City, Guangzhou, 510555 (CN)
(72) Inventor: ZENG, Sumei, Guangzhou, Guangdong 510555 (CN); WANG, Hongxing, Guangzhou, Guangdong 510555 (CN); NING, Xiaohui, Guangzhou, Guangdong 510555 (CN); FAN, Yinzhou, Guangzhou, Guangdong 510555 (CN); ZHAO, Siting, Guangzhou, Guangdong 510555 (CN); QIN, Li, Guangzhou, Guangdong 510555 (CN); CHEN, Xiaoping, Guangzhou, Guangdong 510555 (CN)
(74) Representative: HGF
(86) International application number: PCT/CN2020/121375
(87) International publication number: WO 2021/073586

(57) **Abstract**

Disclosed are a decoquinate liposome, a preparation method therefor and a use thereof. The preparation raw materials for the decoquinate liposome comprise the following components in parts by weight: 1 part of decoquinate and 2-20 parts of lecithin. The liposome is stable, shows favorable malaria resistance effects in-vivo and in-vitro with respect to animal bodies, can be used for quickly relieving the conditions of malaria, particularly subtertian malaria, and is safe. The preparation method is simple and feasible, and has the potential for mass production.

## Description

### TECHNICAL FIELD

The present application belongs to the technical field of medicine and relates to a decoquinate liposome, a preparation method and use thereof and especially, a decoquinate liposome, a preparation method and use thereof.

### BACKGROUND

Malaria is a disease caused by infection with *Plasmodium (P)* parasites introduced by the bite of *Anopheles* mosquitoes. *Plasmodium* parasites such as *P. vivax, P. falciparum, P. malariae* and *P. ovale* infect humans. A parasite, *Plasmodium knowlesi,* infects both humans and monkeys. The most common infections are *falciparum* malaria and *vivax* malaria. Most common clinical manifestations of malaria are fever, headaches, and sweating. In severe cases, malaria can cause asthenia and vomiting. *Falciparum* malaria has a rapid onset and serious conditions, which can lead to severe malaria, cerebral malaria, anemia, multiple organ dysfunction and the highest mortality rate.

*Falciparum* malaria requires prompt treatment; otherwise, it is life-threatening. Due to the widespread problems that chloroquine resistance and sulfonamides resistance of *P. falciparum* emerged in the 1950s and 1960s and then artemisinin resistance, the World Health Organization (WHO) explicitly requires that artemisinin category drugs, the first-line treatment, could not be used alone but must be used in combination with other antimalarial drugs to deal with and prevent the artemisinin resistance. Nevertheless, artemisinin resistance has still emerged in recent years, and there is even an increase of the *Plasmodium* resistance to drugs used in the combination therapy in some areas. Therefore, it is urgent for the pharmaceutical industry to develop new and efficient antimalarials with no known drug resistance.

Decoquinate (CAS No. 18507-89-6) is a quinolone coccidiostat, also known as Deccox. The chemical nomenclature is ethyl 6-decyloxy-7-ethoxy-4-hydroxyquinoline-3-carboxylate, and the molecular weight 417.55 g/mol. It is practically insoluble in water and slightly soluble in ethanol. Decoquinate has strong activity in inhibiting and killing *Plasmodium* by ting the cytochrome *bc1* complex in the mitochondria of *Plasmodium,* and therefore is a potential candidate drug for preventing and treating malaria. Decoquinate not only has a potent activity against the liver stage *Plasmodium,* but also is highly effective against the blood stage *Plasmodium* through the administration of nanoparticle formulations. It can also suppress gametophytes present in the blood. Thereby it may play a role in effectively treating malarial infection and preventing malaria transmission (Nam T-G, et.al, A Chemical Genomic Analysis of Decoquinate, a Plasmodium falciparum Cytochrome b Inhibitor, ACS Chem. Biol. 2011, 6, 1214-1222) (Da Cruz FP, et.al, Drug Screen Targeted at Plasmodium Liver Stages Identifies a Potent Multistage Antimalarial Drug, J. Infect. Dis. 2012, 205, 1278). Furthermore, decoquinate has been used as veterinary medicine for more than 30 years (Taylor M, et.al, The history of decoquinate in the control of coccidial infections in ruminants, J. Vet. Pharmacol. Therap. 2012, 35, 417), which proves that its safety should not be a concern. However, decoquinate has a poor water solubility and bioavailability. Without solving these problems, its development as an antimalarial drug of new generation for human use will be out of the question.

The inventors of this patent work (the same research institution) previously created an oral formulation of decoquinate by solid dispersion. The pharmacodynamic experiments showed that the oral formulation made via decoquinate solid dispersion effectively inhibited *Plasmodium* at the liver stage (Wang Hongxing, et al, Decoquinate solid dispersion, method for preparing same and use thereof , PCT/CN 2015/096689), but did not inhibit the parasites effectively at the erythrocytic stage. Patients with *Plasmodium* infection at the blood stage experience clinical symptoms of acute malaria such as chills, high fever, and anemia. Without prompt treatment, the patients can get serious ill such as cerebral malaria and extremely high fever malaria, even become life threatening.

Therefore, it is of great significance to develop a formulation that can improve the water solubility and bioavailability of decoquinate, meanwhile, to retain the potent and rapid activity against the blood stage *Plasmodium.*

### SUMMARY

The present application provides a decoquinate liposome, a preparation method and use thereof. The liposome formulation improves the water solubility and bioavailability of decoquinate and retains the high potency of its activity and may have a promising application in the future to treat patients with blood stage *Plasmodium* infection and may potentially save the lives of critically ill patients.

In a first aspect, the present invention provides a decoquinate liposome. The components for the decoquinate liposomes include the following by weight: one part of decoquinate and 2-20 parts of lecithin.

The decoquinate liposomes prepared in the present invention has a particle size of diameter 100-200 nm, high encapsulation efficiency and stability. The liposomes have been demonstrated to have highly effective activity against *Plasmodium in vivo* and *in vitro.* Animal studies showed that DQ liposomes could be used to quickly relieve the conditions of malaria, particularly *falciparum* malaria, showed to be safe, did not cause toxic and side effects in mice and hemolytic reaction, and met the requirements of injection preparations.

When the part by weight of the decoquinate is 1 part, the parts by weight of the lecithin may be 2, 4, 5, 8, 10, 12, 14, 16, 18, 20 parts.

Preferably, compositions of the decoquinate liposomes further include the following components in parts by weight: 0.01-5 parts of stabilizer or 0.01-50 parts of surfactant.

The stabilizer or surfactant added in the present application needs to have a specific proportion with the drug with respect to parts by weight, to achieve the maximal efficacy and stability of liposomes.

When the part by weight of the decoquinate is 1 part, the parts by weight of the stabilizer may be 0.01, 0.05, 0.1, 0.2, 0.5, 0.8 and 1 part, or may be 2, 3, 4 and 5 parts

When the part by weight of the decoquinate is 1 part, the parts by weight of the surfactant may be 0.01, 0.05, 0.1, 0.2, 0.5, 0.8 and 1 part, or 2, 3, 4, 5, 8, 10, 12, 15, 20, 25, 30, 40 and 50 parts.

Preferably, compositions of the decoquinate liposomes include the following components in parts by weight: one part of decoquinate, 2-4 parts of lecithin, 0.01-0.5 part of stabilizer, and 0.5-12 parts of surfactant.

Preferably, the lecithin is any one or a combination of at least two of soybean lecithin, egg yolk lecithin, hydrogenated soybean lecithin or hydrogenated egg yolk lecithin. The combination of at least two kinds of the above lecithin is, for example, a combination of soybean lecithin and egg yolk lecithin, a combination of egg yolk lecithin and hydrogenated soybean lecithin, a combination of hydrogenated soybean lecithin and hydrogenated egg yolk lecithin, and other optional combinations are not described in detail herein.

Preferably, the stabilizer includes cholesterol or polyethylene glycol 400.

Preferably, the surfactant includes poloxamer 188 or polyethylene glycol 15-hydroxystearate.

In the present application, with the above-mentioned specific types of stabilizers and surfactant selected to incorporate with active component, the liposome lipid membrane has high deformability, which promotes the transmembrane transport of drugs and improves the pharmacodynamic activity. Meanwhile, the addition of the above-mentioned specific types of stabilizers and surfactants also improves the stability of the product and facilitates clinical application.

The above-mentioned stabilizer and surfactant have been approved by CFDA (China Food and Drug Administration) and FDA (Food and Drug Administration) as excipients for clinical injection.

In a second aspect, the present application provides a method for preparing the above-mentioned decoquinate liposomes. The preparation method has following steps:
(1) decoquinate, lecithin and anhydrous ethanol are mixed and heated in a boiling water bath to obtain an organic phase.
(2) the organic phase mixture obtained in step one is injected into water as the aqueous phase to obtain the decoquinate liposomes.

In the present application, the decoquinate with extremely poor water solubility is encapsulated in liposomes by using ethanol injection method, which enhances the solubility of the decoquinate; and only a small amount of anhydrous ethanol is used in the process, which avoids the problem of solvent toxicity. The preparation method is simple, feasible and has the potential for mass production.

Preferably, when compositions of the liposomes include poloxamer 188 or cholesterol, the poloxamer 188 or cholesterol is added into the organic phase together with decoquinate and lecithin. When compositions of the liposomes include polyethylene glycol 400 or polyethylene glycol 15-hydroxystearate, the polyethylene glycol 400 or polyethylene glycol 15-hydroxystearate is added into the aqueous phase.

Preferably, in step one, the organic mixture is heated for 1-30 minutes until all components are dissolved, for example, 1 minute, 5 minutes, 10 minutes, 12 minutes, 15 minutes, 18 minutes, 20 minutes, 25 minutes, 30 minutes.

Preferably, in step one, a ratio of mass/volume (decoquinate to the anhydrous ethanol) is 0.1-10 mg/mL, for example, 0.1 mg/mL, 0.5 mg/mL, 1 mg/mL, 1 mg/mL, 3 mg/mL, 5 mg/mL, 8 mg/mL, 10 mg/mL.

Preferably, in step two, when the organic phase is injected into the aqueous phase, the aqueous phase is stirred at a speed of 200-2000 rpm, for example, 200 rpm, 300 rpm, 400 rpm, 500 rpm, 800 rpm, 1000 rpm, 1200 rpm, 1400 rpm, 1800 rpm, 2000 rpm.

Preferably, in step two, a volume ratio of the organic phase to the aqueous phase is 1:5-50, for example, 1:5, 1:10, 1:15, 1:20, 1:25, 1:30, 1:35, 1:40, 1:50.

Preferably, in step two, after the organic phase is injected into the aqueous phase, the organic phase and the aqueous phase are continuously stirred for 2-5 minutes, for example, 2 minutes, 2.5 minutes, 3 minutes, 3.5 minutes, 4 minutes, 4.5 minutes, 5 minutes.

The organic phase is injected into the aqueous phase by means of a pipette or a syringe. When the volume of the organic phase is greater than 30 mL, the organic phase is pumped into the aqueous phase at a constant speed by means of a constant flow pump and a hose with a diameter of 1-2 mm, and the speed of the pump is 50-200 rpm.

The common preparation methods of liposomes include film dispersion, cross-film extrusion, high-pressure homogenization, freeze drying and so on. Because of the complex preparation process, poor repeatability, high cost and difficulty in imitation, most methods are only used at the level of laboratory research. In addition, due to the difficulty in the large-scale production of preparation processes and the high investment risk, the industrialization of liposomes is full of challenges, and there is still only a handful of liposome products on the market in China. In the present application, the ethanol injection method is applied to the preparation of decoquinate liposomes, and the preparation process is simple and may be readily used in industrial production.

In a third aspect, the present application provides decoquinate liposome lyophilized powder obtained by lyophilizing the above-mentioned decoquinate liposomes.

In a fourth aspect, the present application provides a preparation method for the above-mentioned decoquinate liposome lyophilized powder. The preparation method includes steps: concentration of decoquinate liposomes, sterile filtration, addition of a protective agent and vacuum lyophilizing decoquinate liposomes to obtain the decoquinate liposome lyophilized powder.

Preferably, the liposomes are concentrated by using a polyether sulfone membrane with a molecular weight cut-off of 5-500 kD, for example, 5 kD, 10 kD, 50 kD, 80 kD, 100 kD, 200 kD, 400 kD, 500 kD.

Preferably, a 0.22 µm filtration membrane is used for sterile filtration.

Preferably, the protective agent includes sucrose and/or trehalose.

Preferably, a mass ratio of the protective agent to lecithin is (1-5):1, for example, 1:1, 1:2, 1:3, 1:4, 1:5.

Preferably, the vacuum lyophilizing lasts for 24-48 hours, for example, 24 hours, 26 hours, 30 hours, 32 hours, 36 hours, 40 hours, 42 hours, 48 hours.

In a fifth aspect, the present application provides the use of the above-mentioned intravenous decoquinate liposomes or the above-mentioned decoquinate liposome lyophilized powder in the preparation of an anti-malarial drug.

In the present application, the intravenous decoquinate liposomes or the decoquinate liposome lyophilized powder can be hydrated and reconstituted, which may be used for, but not limited to, intravenous injection for the treatment of *falciparum* malaria, *vivax* malaria, *oval* malaria, and *knowlesi* malaria.

In a sixth aspect, the present application provides a method for using the above-mentioned intravenous decoquinate liposome lyophilized powder, which includes reconstitution of the decoquinate liposome lyophilized powder with a 5% glucose solution for later use as injection dosage form for the patient.

Compared with the existing art, the present disclosure has beneficial effects described below.

The decoquinate liposomes provided in the present application have a mean particle size of 100-200 nm (diameter), high encapsulation efficiency, reliable stability, potent antimalarial activity *in vivo* and *in vitro.* In animal experiments, the liposomes are used in intravenous injection to quickly relieve the conditions of malaria, particularly *falciparum* malaria, are safe, do not cause toxic and adverse effects in mice and hemolytic reaction, and meet the requirements of injection preparations.

In the present application, decoquinate with extremely poor water solubility is encapsulated in liposomes by ethanol injection method, which enhances the solubility of decoquinate; only a small amount of anhydrous ethanol is used in the preparation process, which avoids the problem of solvent toxicity. The preparation method is simple and feasible and has the potential for mass production.

### BRIEF DESCRIPTION OF DRAWINGS

FIG. 1 is a graph of particle size distribution of liposomes prepared in Examples 4 to 6.
FIG. 2 is a graph of particle size distribution of the liposomes prepared in Example 4 after concentration and resuspension.
FIG. 3 is a flow diagram of the test method of Example 16.
FIG. 4 is a statistical graph of the infection rate of the red blood cells.
FIG. 5 is a statistical graph of the negative conversion ratio.
FIG. 6 is a statistical graph of the survival rate of mice.

### DETAILED DESCRIPTION

The technical solutions of the present application are further described below through the examples. Those skilled in the art are to understand that examples described herein are merely used for a better understanding of the present application and are not to be construed as specific limitations to the present application.

Substances used in the following examples and the sources thereof are as follows:
Decoquinate (batch No.: 130802, molecular weight: 417.53 g/mol, produced by Zhejiang
Genebest Pharmaceutical Co., Ltd.).
Soybean lecithin (injection grade, Shanghai Tywei Pharmaceutical Co., Ltd.).
Egg yolk lecithin (injection grade, produced by Guangzhou Bai Yun Shan Hanfang Modern Pharmaceutical Co., Ltd.).
Hydrogenated soybean lecithin (injection grade, produced by Guangzhou Bai Yun Shan Hanfang Modern Pharmaceutical Co., Ltd.).
Poloxamer 188 (Kolliphor^{®} P188, BASF, Germany).
Polyethylene glycol 400 (injection grade, Jiangxi Yi Pu Sheng Pharmaceutical Co., Ltd).
Polyethylene glycol 15-hydroxystearate (Kolliphor HS 15, BASF, Germany).
Cholesterol (injection grade, Guangzhou Bai Yun Shan Hanfang Modern Pharmaceutical Co., Ltd.).

### Example 1

This example provides a decoquinate liposome. The preparation method of the decoquinate liposomes has the following steps.
(1) Twenty mg of decoquinate, 80 mg of soybean lecithin, 15 mL of anhydrous ethanol were weighed, and 285 mL of water measured.
(2) The above decoquinate and soybean lecithin were added to the anhydrous ethanol, and then heated in a boiling water bath for 10 minutes or until decoquinate and soybean lecithin were dissolved; the mixture was taken as an organic phase.
(3) An aqueous phase water) was magnetically stirred at a speed of 500 rpm, the organic phase obtained in step two was slowly injected into the aqueous phase by means of a pipette, where the volume ratio of the organic phase to the aqueous phase was 1:19, and the stirring was continued for 2 min after all the organic phase was injected into the aqueous phase to obtain the decoquinate liposomes.

### Example 2

This example provides a decoquinate liposome. The preparation method of the decoquinate liposomes has following steps.
(1) Twenty mg of decoquinate, 40 mg of soybean lecithin, 10 mg of cholesterol, 30 mL of anhydrous ethanol were weighed, and 270 mL of water measured.
(2) The above decoquinate, soybean lecithin and cholesterol were added to the anhydrous ethanol, and then heated in a boiling water bath for 10 minutes or until decoquinate, soybean lecithin and cholesterol were dissolved; the mixture was as an organic phase.
(3) Water as an aqueous phase was magnetically stirred at a speed of 1000 rpm, the organic phase obtained in step two was slowly injected into the aqueous phase by means of a pipette, where a volume ratio of the organic phase to the aqueous phase was 1:9, and the stirring was continued for 5 minutes after all the organic phase was injected into the aqueous phase to obtain the decoquinate liposomes.

### Example 3

This example provides a decoquinate liposome. The preparation method of the decoquinate liposomes has following steps.
(1) Forty mg of decoquinate, 80 mg of soybean lecithin, 130 mg of polyethylene glycol 15-hydroxystearate, 30 mL of anhydrous ethanol were weighed, and 270 mL of water measured.
(2) The above decoquinate and soybean lecithin were added to the anhydrous ethanol, and then heated in a boiling water bath for 10 minutes or until decoquinate and soybean lecithin were dissolved; the mixture was as an organic phase.
(3) With water as an aqueous phase, polyethylene glycol 15-hydroxystearate was added to water, the aqueous phase was magnetically stirred at a speed of 200 rpm, the organic phase obtained in step two was slowly injected into the aqueous phase by means of a pipette, where a volume ratio of the organic phase to the aqueous phase was 1:9, and the stirring was continued for 5 minutes after all the organic phase was injected into the aqueous phase to obtain the decoquinate liposomes.

### Example 4

This example provides a decoquinate liposome. The preparation method of the decoquinate liposomes has following steps.
(1) Twenty mg of decoquinate, 80 mg of soybean lecithin, 160 mg of poloxamer 188, 65 mg of polyethylene glycol 400, 15 mL of anhydrous ethanol were weighed, and 285 mL of water accurately measured.
(2) The above decoquinate, soybean lecithin and poloxamer 188 were added to the anhydrous ethanol, and then heated in a boiling water bath for 10 minutes or until decoquinate, soybean lecithin and poloxamer 188 were dissolved; the mixture was as an organic phase.
(3) With water for injection as an aqueous phase, the polyethylene glycol 400 was added to the water for injection, the aqueous phase was magnetically stirred at a speed of 500 rpm, the organic phase obtained in step two was slowly injected into the aqueous phase by means of a pipette, where a volume ratio of the organic phase to the aqueous phase was 1:19, and the stirring was continued for 5 minutes after all the organic phase was injected into the aqueous phase to obtain the decoquinate liposomes.

### Example 5

This example provides a decoquinate liposome. The preparation method of the decoquinate liposomes has following steps (the formulation scale of Example 4 was increased by five times).
(1) A hundred mg of decoquinate, 400 mg of soybean lecithin, 900 mg of poloxamer 188, 325 mg of polyethylene glycol 400, 75 mL of anhydrous ethanol were weighed, and 1.25 L of water accurately measured.
(2) The above decoquinate, soybean lecithin and poloxamer 188 were added to the anhydrous ethanol, and then heated in a boiling water bath for 15 minutes or until decoquinate, soybean lecithin and poloxamer 188 were dissolved; the mixture was as an organic phase.
(3) With water for injection as an aqueous phase, the polyethylene glycol 400 was added to the water for injection, the aqueous phase was magnetic stirred at a stirring speed of 1000 rpm, the organic phase obtained in step two was slowly injected into the aqueous phase by means of a constant flow pump and a hose with a diameter of 1 mm, where a volume ratio of the organic phase to the aqueous phase was 1:17, and the stirring was continued for 5 minutes after all the organic phase was injected into the aqueous phase to obtain the decoquinate liposomes.

### Example 6

This example provides a decoquinate liposome. The preparation method of the decoquinate liposomes has following steps (the formulation of Example 4 was proportionally scaled up to ten times).
(1) Two hundred mg of decoquinate, 800 mg of soybean lecithin, 1.6 g of poloxamer 188, 650 mg of polyethylene glycol 400, 150 mL of anhydrous ethanol were weighed, and 2.5 L of water accurately measured.
(2) The above decoquinate, soybean lecithin and poloxamer 188 were added to the anhydrous ethanol, and then heated in a boiling water bath for 20 minutes or until decoquinate, soybean lecithin and poloxamer 188 were dissolved; the mixture was as an organic phase.
(3) With water as an aqueous phase, the polyethylene glycol 400 was added to the water, the aqueous phase was magnetically stirred at a speed of 2000 rpm, the organic phase obtained in step 2 was slowly injected into the aqueous phase by means of a constant flow pump and a hose with a diameter of 2 mm, where a volume ratio of the organic phase to the aqueous phase was 1:17, and the stirring was continued for 5 minutes after all the organic phase was injected into the aqueous phase to obtain the decoquinate liposomes.

### Example 7

This example provides a decoquinate liposome. The preparation method of the decoquinate liposomes has following steps.
(1) Twenty mg of decoquinate, 80 mg of egg yolk lecithin, 15 mL of anhydrous ethanol were weighed, and 285 mL of water accurately measured.
(2) The above decoquinate and egg yolk lecithin were added to the anhydrous ethanol, and then heated in a boiling water bath for 10 minutes or until decoquinate and egg yolk lecithin were dissolved; the mixture was as an organic phase.
(3) An aqueous phase was magnetically stirred at a speed of 500 rpm, the organic phase obtained in step two was slowly injected into the aqueous phase by means of a pipette, where a volume ratio of the organic phase to the aqueous phase was 1:19, and the stirring was continued for 2 minutes after all the organic phase was injected into the aqueous phase to obtain the decoquinate liposomes.

### Example 8

This example provides a decoquinate liposome. The preparation method of the decoquinate liposomes has following steps.
(1) Twenty mg of decoquinate, 80 mg of hydrogenated soybean lecithin, 15 mL of anhydrous ethanol were weighed, and 285 mL of water accurately measured.
(2) The above decoquinate and hydrogenated soybean lecithin were added to the anhydrous ethanol, and then heated in a boiling water bath for 10 minutes or until decoquinate and hydrogenated soybean lecithin were dissolved; the mixture was as an organic phase.
(3) An aqueous phase was magnetic stirred at a stirring speed of 500 rpm, the organic phase obtained in step two was slowly injected into the aqueous phase by means of a pipette, where a volume ratio of the organic phase to the aqueous phase was 1:19 and the stirring was continued for 2 minutes after all the organic phase was injected into the aqueous phase to obtain the decoquinate liposomes.

### Example 9

This example provides a decoquinate liposome. The preparation method of the decoquinate liposomes has following steps.
(1) Forty mg of decoquinate, 80 mg of hydrogenated soybean lecithin, 60 mg of poloxamer 188, 102 mg of polyethylene glycol 400, 7 mg of cholesterol, 15 mL of anhydrous ethanol were weighed, and 285 mL of water accurately measured.
(2) The above decoquinate, hydrogenated soybean lecithin, poloxamer 188 and cholesterol were added in the anhydrous ethanol, and then heated in a boiling water bath for 10 minutes or until decoquinate, hydrogenated soybean lecithin, poloxamer 188 and cholesterol were dissolved; the mixture was as an organic phase.
(3) The polyethylene glycol 400 was added to the water, the aqueous phase was magnetic stirred at a stirring speed of 500 rpm, the organic phase obtained in step two was slowly injected into the aqueous phase by means of a pipette, where a volume ratio of the organic phase to the aqueous phase was 1:19, and the stirring was continued for 2 minutes after all the organic phase was injected into the aqueous phase to obtain the decoquinate liposomes.

### Example 10

This example provides a decoquinate liposome. The preparation method of the decoquinate liposomes has following steps.
(1) Forty mg of decoquinate, 80 mg of hydrogenated soybean lecithin, 65 mg of polyethylene glycol 400, 40 mg of cholesterol, 14 mL of anhydrous ethanol were weighed, and 180 mL of water accurately measured.
(2) The above decoquinate, hydrogenated soybean lecithin and cholesterol were added in the anhydrous ethanol, and then heated in a boiling water bath for 15 minutes or until decoquinate, hydrogenated soybean lecithin and cholesterol were dissolved; the mixture was as an organic phase.
(3) The polyethylene glycol 400 was added to water as the aqueous phase and magnetically stirred at a speed of 500 rpm, the organic phase obtained in step two was slowly injected into the aqueous phase by means of a pipette, where a volume ratio of the organic phase to the aqueous phase was 1:13, and the stirring was continued for 2 minutes after all the organic phase was injected into the aqueous phase to obtain the decoquinate liposomes.

### Example 11

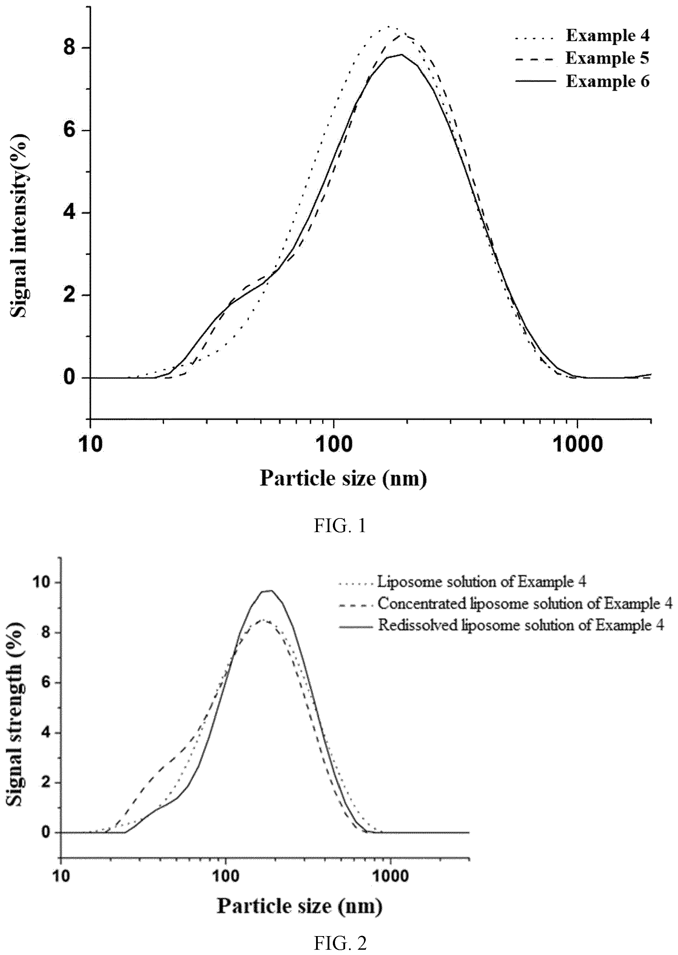

In this example, the stability of liposomes was evaluated. The specific method was as follows. The decoquinate liposome preparations in Examples 1 to 6 were placed at 4°C, and the changes in particle size and encapsulation efficiency on day 0 and day 5 were examined. The average particle size, polydispersity index (PDI) and Zeta potential of liposomes were measured by Malvern Laser Nano Particle Size Analyzer (Zetasizer Nano ZSE, Malvern Panalytical Ltd, UK). The encapsulation efficiency was measured in the following method. The liposome preparation was centrifuged at 5000 rpm for 10 minutes, and then the supernatant was collected and the DQ concentration determined by high performance liquid chromatography (HPLC 1260, produced by Agilent). The formula for calculating the encapsulation efficiency was as follows. The results are shown in Table 1 and Fig. 1.

**Table 1**

| Measurements | Days | Liposome formulations | | | |
|---|---|---|---|---|---|
| | | Example 1 | Example 2 | Example 3 | Example 4 |
| Particle size (nm) | 0 | 141.5 ± 0.3 | 124.7±1.3 | 144.1±4.0 | 133.1±1.4 |
| | 5 | 146.9±2.6 | 153.3±1.1 | 153.6±0.6 | 149.8±0.4 |
| PDI | 0 | 0.266±0.003 | 0.200±0.006 | 0.213±0.006 | 0.269±0.005 |
| | 5 | 0.259±0.001 | 0.194±0.007 | 0.234±0.003 | 0.271±0.021 |
| Zeta potential (mV) | 0 | 0.90±0.17 | 2.21±0.62 | -6.59±0.33 | -17.4±0.59 |
| | 5 | 1.11±0.11 | 5.31±0.34 | -9.76±0.47 | -19.9±0.76 |
| Encapsulation efficiency (%) | 0 | 98.80 | 96.30 | 99.20 | 98.70 |
| | 5 | 99.30 | 95.90 | 99.80 | 99.60 |

As seen from Table 1, the average particle size of liposomes of Examples 1 to 4 on day 0 is less than 150 nm, the PDI lower than 0.3, and the encapsulation efficiency greater than 95%. After liposome preparations were stored at 4°C for 5 days, the encapsulation efficiency remained unchanged, but the average particle size is increased slightly, among which, the average particle size of Example 2 is increased most significantly.

The graph of particle size distribution of liposomes prepared in Examples 4 to 6 is shown in Fig. 1. As can be seen from the graph, the appearance of liposomes with small particle size results in the occurrence of a shoulder in the curve, but the average particle sizes of liposomes of Examples 4 to 6 are 133.1 nm, 140.3 nm and 132.9 nm respectively; the particle sizes are generally the same, and liposome preparations appear to be clear and transparent, which indicates that the preparation process is quite stable and may have the potential for industrial production.

### Example 12

In this example, the stability of liposomes in the process of concentrating, lyophilizing and lyophilized powder storage was evaluated. The specific method was as follows:

### (1) Concentration and lyophilization

The decoquinate liposome preparations in Examples 1 to 4 were concentrated, lyophilized, and the changes in both particle size and encapsulation efficiency of liposomes were assessed in the following steps:
(A) The liposome preparation was concentrated to 2 mg/mL of decoquinate by a tangential flow filtration system, where the hollow fiber membrane used was made of polyether sulfone with a size of 500 kD.
(B) The concentrated liposomes in step A were filtered to sterilized liposomes by using a 0.22 µm filtration membrane, to which sucrose was then added, and the resulting mixture was aliquoted in vials, vacuum lyophilized for 48 hours, gassed with nitrogen and sealed to obtain the decoquinate liposome lyophilized powder, where a mass ratio of sucrose to phospholipid was 4:1.

Changes of the average particle size of the liposomes of Examples 1 to 4 during the concentration process are shown in Table 2.

**Table 2**

| Samples | Before concentration (nm) | After concentration (nm) |
|---|---|---|
| Example 1 | 141.5 ± 0.3 | 205.7 ±4.6 |
| Example 2 | 124.7±1.3 | Floccule aggregation |
| Example 3 | 144.1±4.0 | 127.0±1.0 |
| Example 4 | 133.1±1.4 | 130.0±3.8 |

As seen from the data in Table 2, the liposomes prepared in Examples 3 and 4 have minor change in particle size during concentration, which means that the obtained liposomes as such are more stable.

The decoquinate liposome lyophilized powder prepared in Examples 3 and 4 was reconstituted with 5% glucose solution, and the average particle size and encapsulation efficiency of the decoquinate liposomes are shown in Table 3.

**Table 3**

| Sample | Average particle size (nm) | Encapsulation efficiency (%) |
|---|---|---|
| Example 3 | 153.4±3.9 | 96.1±1.5 |
| Example 4 | 152.9±2.2 | 95.9±1.2 |

As seen from the data in Table 3, the average particle size of the decoquinate liposomes included in the present application is less than 200 nm, and the encapsulation efficiency remained above 95%.

Shown in Fig. 2 are effects of the concentration and reconstitution process of the liposomes prepared in Example 4 on the particle size distribution. As seen from Fig. 2, the concentration process makes the particle size distribution of the liposomes slightly wider, but after the lyophilized liposomes were resuspended with 5% glucose solution, the liposomes have narrower particle size distribution and better particle size uniformity.

### (2) Storage of lyophilized powder

The decoquinate liposome lyophilized powder prepared in Examples 3 and 4 was stored at -20°C, and the changes in particle size and encapsulation efficiency were examined. The results are shown in Table 4.

**Table 4**

| Month | Particle size (nm) | | PDI | | Encapsulation efficiency (%) | |
|---|---|---|---|---|---|---|
| | 0 | 6 | 0 | 6 | 0 | 6 |
| Example 3 | 153.4±3.9 | 155.9±1.5 | 0.279±0.004 | 0.262±0.002 | 96.7 | 96.7 |
| Example 4 | 152.9±2.2 | 161.5±1.0 | 0.280±0.001 | 0.286±0.029 | 96.4 | 95.8 |

As can be seen from Table 4, the particle size and encapsulation efficiency of decoquinate liposomes were not significantly changed after the lyophilized powder produced from the decoquinate liposomes was stored for 6 months, indicating that the decoquinate liposomes of Examples 3 and 4 were quite stable.

### Example 13

In this example, the hemolytic effects of liposomes have been evaluated, and the specific method is as follows.
(1) Preparation of 2% red blood cell suspension. Two mL blood of healthy volunteer was centrifuged at 3000 rpm for 10 minutes, and the supernatant was discarded; erythrocytes were washed with normal saline 3 times until the wash solution became colorless and transparent, and the washed red blood cells were diluted with normal saline into 2% red blood cell suspension for later use.
(2) Experimental design. Six clean test tubes labeled as test tubes 1 and 2 had 1 mg/mL liposomal DQ of Example 4 whereas test tubes 3 and 4 had 1 mg/mL liposomal DQ of Example 3; test tube 5 was used as a negative control and test tube 6 as a positive control tube. To all tubes solutions shown in Table 5 were added respectively, shaken well, and incubated at 37°Cfor 3 hours. One and half mL of supernatant was transferred to a new tube and centrifuged at 3000 rpm for 10 minutes. Then 1 mL of supernatant was transferred to a centrifuge tube, to which 0.1 mL of 10% Triton-X100 was added. Two hundred µL of each resulting mixture were used to be measured at 540 nm absorbance.
(3) The hemolysis rates were calculated according to the following formula: hemolysis rate (%) = (Aₛₐₘₚₗₑ - Aₚₒₛᵢₜᵢᵥₑ)/ (A_{negative} - Aₚₒₛᵢₜᵢᵥₑ) × 100%.

**Table 5**

| Test tubes | 1 | 2 | 3 | 4 | 5 | 6 |
|---|---|---|---|---|---|---|
| Liposomes (mL) | 0.3 | 0.3 | 0.3 | 0.3 | / | / |
| Normal saline (mL) | 2.2 | 2.2 | 2.2 | 2.2 | 2.5 | / |
| Distilled water (mL) | / | / | / | / | / | 2.5 |
| RBC suspension (mL) | 2.5 | 2.5 | 2.5 | 2.5 | 2.5 | 2.5 |
| Absorbance | 0.057 | 0.053 | 0.067 | 0.069 | 0.048 | 1.081 |

The results show that the hemolysis rate of the liposomes of Example 4 was 0.73%, and the hemolysis rate of the liposomes of Example 3 was 2.03%, both of which were lower than 5%, which indicated that the decoquinate liposomes prepared by this method did not cause hemolytic reaction and met the requirements of intravenous injection formulation.

### Example 14

In this example, the acute toxicity of liposomes in mice is assessed, and the specific method is as follows.

Twenty-four Kunming mice weighing 20-25 g were randomly divided into three groups: experimental group 1, experimental group 2, and control group, with half male and half female in each group. The mice in the experimental group 1 were injected with the decoquinate liposomes prepared in Example 4 via the tail vein, and the mice in the experimental group 2 were injected with the decoquinate liposomes prepared in Example 3 via the tail vein, with the dosage of the liposomal decoquinate of 500 mg/kg; the mice in the control group were given the same amount of liposomes free of decoquinate. The mice were observed for 14 days for acute toxicity such as piloerection, respiratory rate change, body surface color change, salivation, vomiting, seizure, and convulsion.

The mice in experimental group 1, experimental group 2 and control group were observed for 14 days. The results show that no difference in behavior was found, which indicated that the decoquinate liposomes had no obvious toxic and adverse effects on mice when the dosage was as high as 500 mg/kg.

### Example 15

In this example, in-vitro inhibition potency of liposomes has been assessed, and the specific method is as follows.
(1) Culture medium: 10.43 g of 1640 dry powder (GIBCO, 31800022), 5.96 g of Hepes, 2 g of sodium bicarbonate, 0.292 g of L-glutamine, 3.96 g of glucose, 50 mg of hypoxanthine, 10 mg of gentamicin and 5 g of AlbumaxI/II (GIBCO, A1049101) were placed in a 1 L beaker containing proper amount of ultrapure water, and magnetically stirred until the above materials were completely dissolved. The volume was filled up to 1 L with ultrapure water, the pH adjusted to 7.2, subjected to sterilized filtration by using a 0.22 µm filtration membrane, and stored at 4°C to obtain a complete culture medium; the incomplete medium was composed of 10.43 g of 1640 dry powder (GIBCO, 31800022), 5.96 g of Hepes, 2 g of sodium bicarbonate, 0.292 g of L-glutamine, 3.96 g of glucose, 50 µg of hypoxanthine and 2.5 mg of gentamicin, prepared by the same method as for the complete culture medium.
(2) Drugs and formulations: pure decoquinate was dissolved in DMSO as a stock solution, chloroquine was dissolved in PBS, and artemisinin was dissolved in DMSO and then diluted with PBS. The liposomes evaluated were samples prepared in Examples 1 to 4, all of which were prepared with the incomplete culture medium used for parasitized red blood cells. Before the experiment, all the above dissolved drug solutions were then diluted to series of 10 gradient concentrations with the incomplete medium.
(3) Culture and assay method: *Plasmodium falciparum* was cultured in the complete culture medium, and human red blood cells (RBC) were preserved in the incomplete culture medium. A 96-well plate used for the experiment had 10 µL of drug and 90 µL of *Plasmodium* blood in each well (the standard of *Plasmodium* blood was 0.5% infection rate and 2% hematocrit), a total volume of 100 µL in each well of the culture. The culture was incubated at 37°C in 5% CO₂ for 72 hours, then nucleic acid dye was added, and the absorbance measured in a microplate reader. The half-maximal inhibitory concentration (IC₅₀) of drugs was calculated with Graph Pad software, that is, the concentration when the inhibition rate of antimalarial drugs to *P. falciparum* reached 50% was calculated.

IC₅₀ values of pure decoquinate (DQ), chloroquine (CQ), artemisinin (Art) and the prepared liposomes against *P. falciparum* are shown in Table 6, in which *P. falciparum* 3D7 was a chloroquine-sensitive strain and *P. falciparum* Dd2 a multidrug-resistant (including chloroquine) strain. The smaller the IC₅₀, the stronger the inhibitory effect of drugs on *Plasmodium.*

**Table 6**

| Test sample | IC₅₀ | |
|---|---|---|
| | *P. falciparum* 3d7 Mean±S.D. (nM) | *P. falciparum* Dd2 Mean±S.D. (nM) |
| Example 1 | 2.47±0.23 | / |
| Example 2 | 2.11±0.52 | / |
| Example 3 | 1.67±0.04 | 1.12±0.11 |
| Example 4 | 0.91±0.05 | 1.33±0.14 |
| Pure DQ | 1.63±0.13 | 1.57±0.49 |
| Chloroquine | 23.75±0.06 | 184.8±16.24 |
| Artemisinin | 16.44±5.80 | 18.53±0.50 |

As shown in the table, nM represents nanomolar concentration and IC₅₀ values of the four liposomes are the decoquinate concentrations. As can be seen in Table 6, the decoquinate liposomes prepared in Example 4 had stronger inhibitory effect on *Plasmodium falciparum* than pure decoquinate dissolved in DMSO, whose IC₅₀ for the chloroquine-sensitive strain *P. falciparum* 3D7 was only 1/18 of that of the first-line antimalarial drug artemisinin. Compared with artemisinin, the liposomes of Example 3 and Example 4 showed more efficient inhibitory effects on the multidrug-resistant strain *P. falciparum* Dd2.

### Example 16

In this Example, in-vivo efficacy of liposomes in the blood stage was assessed, and the specific method is as follows.

The experiment was performed in strict accordance with the relevant animal experiment regulations (REGULATIONS AFFAIRS CONCERNING EXPERIMENTAL ANIMALS, approved by the State Council of China, revised version 2017.3). The doses described below refer to the concentrations calculated according to decoquinate.

### (1) Experiment material

Experimental animals: Kun Ming mice, weighed about 30 g, randomly divided into 7 groups, with 10 mice per group.

*Plasmodium* strain: *P. berghei* ANKA 868, the first-generation strain obtained after the passage isolation from mosquitos.

Drugs: decoquinate liposomes prepared in Example 4, diluted with normal saline before injection.

### (2) Experimental method

Mice were divided into a normal saline group and decoquinate liposome groups in which mice were given 0.04 mg/kg, 0.12 mg/kg, 0.37 mg/kg, 1.11 mg/kg, 3.33 mg/kg, and 10.0 mg/kg, respectively.

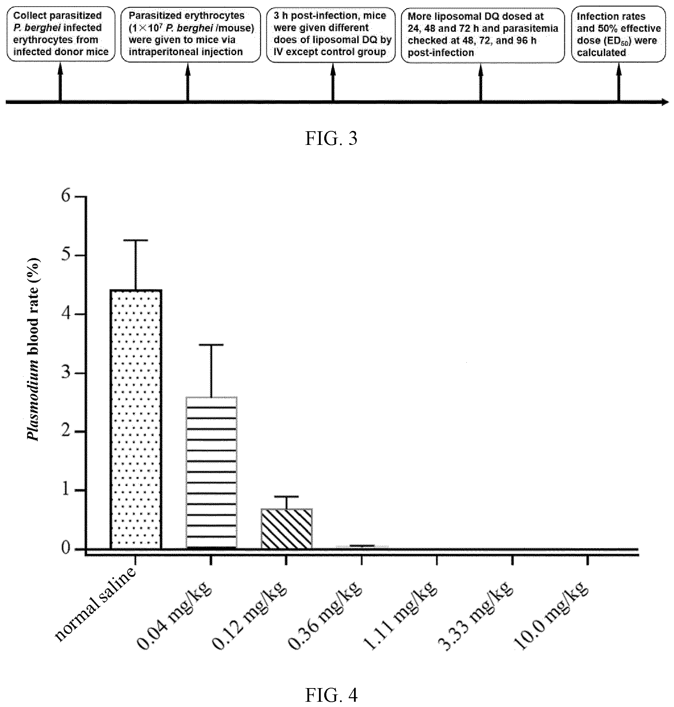

Experimental time course is illustrated as shown in Fig. 3. Mice in each group were infected by the *Plasmodium* as follows. Each mouse was intraperitoneally injected with *Plasmodium* infected red blood cells, where the amount of injected *Plasmodium* was 1×10⁷ per mouse. The mice were injected with liposomal decoquinate at 3 hours, 24 hours, 48 hours and 72 hours via the tail vein after the mice were inoculated with the *Plasmodium.* Blood smears were prepared from the blood drawn from the tail vein, starting at 48 hours and 72 hours for examination. Blood smears were prepared from the blood drawn from the tail vein were examined 24 hours after the last administration of DQ liposomes, the 50% negative conversion rate (ED₅₀) calculated, and the blood smears examined every 3 days after drug the DQ liposomes ended and lasted for 30 days.

### (3) Experimental results

### (A) Red blood cell infection statistics and ED₅₀

Twenty-four hours after the last administration of DQ liposomes, the blood smears taken via the tail vein were examined, and the red blood cell infection rate counted. The results are shown in Fig. 4. As can be seen from the figure, the low dose of liposomal decoquinate had a significantly inhibitory effect on the growth of *Plasmodium.*

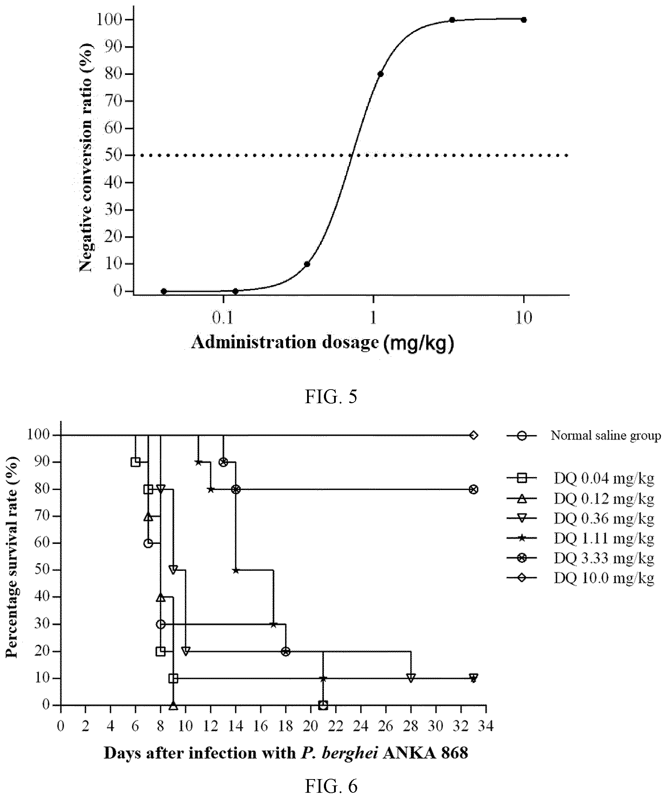

The results of 4-day inhibition experiments of decoquinate liposomes are shown in Fig. 5, and as can be seen from the figure, ED₅₀ of the liposomal decoquinate in mice was 0.7195 mg/kg.

### (B) Relapse occurrence

Blood smears taken via the tail vein were examined 33 days after the infection (30 days after drug dose ended). As shown in Table 7, 2 of the 10 mice in the liposomal decoquinate 3.33 mg/kg group had recrudescent malaria and died whereas none of the 10 mice in the liposomal decoquinate 10 mg/kg group had recrudescent infection and the mice were all in good condition. The number of infections was the results of the 4-day inhibition experiment, in which if red blood cells were infected through the smear examination, the mice were regarded as being infected while if no red blood cell was infected, the *Plasmodium* infection in mice was regarded as being inhibited. The number of recrudescent infections was the results of the experiment 30 days after drug dose ended, in which if no infection appeared within 4 days after the liposomal decoquinate administration but then infection was detected, the mice were regarded as being recrudescent.

**Table 7**

| Liposomal DQ (mg/kg) | Control 0 | Experimental groups | | | | | |
|---|---|---|---|---|---|---|---|
| | | 0.04 | 0.12 | 0.36 | 1.11 | 3.33 | 10 |
| Number of mice | 10 | 10 | 10 | 10 | 10 | 10 | 10 |
| Number of infected mice | 10 | 10 | 10 | 9 | 2 | 0 | 0 |
| Number of non-infected mice | 0 | 0 | 0 | 1 | 8 | 10 | 10 |
| Number of relapsed mice | / | / | / | 9 | 8 | 2 | 0 |
| Relapse rate (%) | / | / | / | 90 | 80 | 20 | 0 |

### (c) Mice survival rate

The mice survival rates are shown in Fig. 6. On day 33 of infection (30 days after drug dose ended), mice in the normal saline group, 0.04 mg/kg decoquinate liposome group and 0.12 mg/kg decoquinate liposome group all died, one mouse in the 0.36 mg/kg decoquinate liposome group and one mouse in the 1.11 mg/kg decoquinate liposome group survived, eight mice in the 3.33 mg/kg decoquinate liposome group survived, and 10 mice in the 10 mg/kg decoquinate liposome group all survived. The growth of *Plasmodium* was completely inhibited in mice administered at liposomal decoquinate dose of 10 mg/kg and the survival rates were100%.

The applicant has stated that although the decoquinate liposomes, a preparation method and use thereof in the present application are described through the examples described above, the present application is not limited to the examples described above, which means that implementation of the present application does not necessarily depend on the examples described above. It should be apparent to those skilled in the art that any improvements made to the present application, equivalent replacements of components of the product of the present application, additions of adjuvant ingredients to the product of the present application, and selections of specific manners, etc., all fall within the protection scope and the disclosed scope of the present application.

Though the preferred embodiments of the present application have been described above in detail, the present application is not limited to details of the above-described embodiments, and various simple modifications can be made to the technical solutions of the present application without departing from the scope of the present application. These simple modifications are all within the protection scope of the present application.

In addition, it is to be noted that if not in collision, the specific technical features described in the above specific embodiments may be combined in any suitable manner. To avoid unnecessary repetition, the present application does not further specify any of various combinations.

## Claims

1. A decoquinate liposome, wherein compositions for the decoquinate liposomes comprise the following components in parts by weight, 1 part of decoquinate and 2-20 parts of lecithin.

2. The decoquinate liposomes according to claim 1, wherein the compositions of the decoquinate liposomes further comprise the following components in parts by weight: 0.01-5 parts of stabilizer and/or 0.01-50 parts of surfactant.

3. The decoquinate liposomes according to claim 1 or 2, wherein the compositions of the decoquinate liposomes comprise the following components in parts by weight: 1 part of decoquinate, 2-4 parts of lecithin, 0.01-0.5 part of stabilizer, and 0.5-12 parts of surfactant.

4. The decoquinate liposomes according to any one of claims 1 to 3, wherein the lecithin comprises any one or a combination of at least two of soybean lecithin, egg yolk lecithin, hydrogenated soybean lecithin or hydrogenated egg yolk lecithin.

5. The decoquinate liposomes according to any one of claims 2 to 4, wherein the stabilizer comprises cholesterol and/or polyethylene glycol 400; and/or
the surfactant comprises poloxamer 188 and/or polyethylene glycol 15-hydroxystearate.

6. The decoquinate liposomes according to any one of claims 1 to 5, wherein the particle sizes of the decoquinate liposomes range from 100 nm to 200 nm.

7. A preparation method for the decoquinate liposomes according to any one of claims 1 to 6, comprising following steps:
(1) decoquinate, lecithin and anhydrous ethanol are mixed and heated in a boiling water bath to obtain an organic phase mixture;
(2) with water as an aqueous phase, the organic phase mixture obtained in step one is injected to the aqueous phase to obtain the decoquinate liposomes.

8. The preparation method according to claim 7, wherein in step one, the aqueous phase is heated for 1-30 minutes.

9. The preparation method according to claim 7, wherein in step one, a mass-to-volume ratio of the decoquinate to the anhydrous ethanol is 0.1-10 mg/mL.

10. The preparation method according to claim 7, wherein in step two, when the organic phase is injected into the aqueous phase, the aqueous phase is stirred at a speed of 200-2000 rpm.

11. The preparation method according to claim 7, wherein in step two, a volume ratio of the organic phase to the aqueous phase is 1:(5-50); and/or
in step two, after the organic phase is injected into the aqueous phase, the organic phase and the aqueous phase are continuously stirred for 2-5 minutes.

12. Decoquinate liposome lyophilized powder obtained by lyophilizing the decoquinate liposomes according to any one of claims 1 to 6.

13. A preparation method for the decoquinate liposome lyophilized powder according to claim 12 comprises concentration, sterile filtration, addition of a protective agent and vacuum lyophilizing decoquinate liposomes to obtain the decoquinate liposome lyophilized powder.

14. The preparation method according to claim 13, wherein the concentration is through the process by using a polyether sulfone membrane having a molecular weight cut-off of 5-500 kD;
optionally, a 0.22 µm filtration membrane is used for the sterile filtration;
optionally, the protective agents comprise sucrose and/or trehalose;
optionally, the mass ratio of the protective agent to the lecithin is (1-5):1; and
optionally, the vacuum lyophilizing lasts for 24-48 hours.

15. Use of the decoquinate liposome according to any one of claims 1 to 6 or the decoquinate liposome lyophilized powder according to claim 12 in the preparation of an antimalarial drug.
